# EUROPEAN PATENT APPLICATION

(11) **EP 4 505 951 A1**
(43) Date of publication of application: **12.02.2025**
(21) Application number: 24193044.5
(22) Date of filing: 06.08.2024
(51) Int. Cl.: A61B 17/16

(54) **COUPLING APPARATUS FOR ROTARY SURGICAL TOOL**

(30) Priority: 07.08.2023 US 202363531131 P
(71) Applicant: Arthrex, Inc, Naples, FL 34108-1945 (US)
(72) Inventor: Lombardo, Giuseppe, Naples, FL, 34108 (US); Fritz, Joseph A., Naples, FL, 34108 (US); Deckers, Sebastian, Naples, FL, 34108 (US); Traurig, Austin, Naples, FL, 34108 (US)
(74) Representative: Lohr, Jöstingmeier & Partner

(57) **Abstract**

A coupling interface for a surgical tool includes a shank of an interchangeable tool accessory forming a proximal engaging portion including a receiving groove. A receiving coupler receives the shank and includes a first socket portion and a second socket portion. The socket portions form a receiving profile extending about a rotational engagement axis of a drive head. A locking collar is interposed between the socket portions and a release actuator is in connection with the locking collar. The release actuator includes ramp surface that radially varies about the engagement axis along a ramp angle. A rotation of the release actuator about the engagement axis angularly repositions the ramp surface and displaces the locking collar.

## Description

### CROSS-REFERENCE TO RELATED APPLICATIONS

This application claims priority under 35 U.S.C. § 119(e) and the benefit of U.S. Provisional Application No. 63/531,131 entitled COUPLING APPARATUS FOR ROTARY SURGICAL TOOL, filed on August 7, 2023, by Giuseppe Lombardo, et al., the entire disclosure of which is incorporated herein by reference.

### BACKGROUND

The present disclosure generally relates to an apparatus for the interchangeable attachment of accessories for surgical instruments. More particularly, the disclosure relates to a connection interface for engaging surgical tools configured for rapid exchange and installation.

### SUMMARY

Surgical accessories and tools may be applied to support a wide variety of procedures and surgical operations. As the variety of use cases and applications for such tools increase and diversify, the complexity of operation of the tools may similarly increase. In various implementations, the disclosure provides for a coupling apparatus for a rotary surgical tool that may improve user interaction for professionals with diverse preferences for operation. For example, in some cases, the coupling apparatus may provide for ambidextrous operation by engaging or releasing corresponding drive heads or accessories by either clockwise or counterclockwise rotation. The ambidextrous operation may also provide for the release of drive heads via a single finger engagement (e.g., an index or trigger-finger) that otherwise controls a drive control input of a power tool. The coupling apparatus and associated locking interface may also provide improved tool longevity by limiting wear components that may be damaged or deteriorate over time. Detailed examples of features and corresponding mechanisms of the coupling apparatus are described in reference to exemplary assemblies in the detailed description that follows.

In some implementations, the disclosure provides for a coupling apparatus for a surgical tool. The coupling apparatus may include a receiving coupler comprising first and second socket portions that form a receiving profile aligned with a rotational engagement axis of a drive head. A locking collar may be interposed between the first and second socket portions and a release actuator may be in connection with the receiving coupler. A rotation of the release collar relative to the socket about the engagement axis moves the locking collar relative to the engagement axis. The movement of the locking collar may cause a locking surface of the locking collar to move away from the engagement axis and withdraw from a locking position extending into the receiving profile of the receiving coupler. In this way, the rotation of the release actuator may allow the drive head to be disengaged from and removed from the receiving coupler along the engagement axis.

These and other features, objects and advantages of the present disclosure will become apparent upon reading the following description thereof together with reference to the accompanying drawings.

### BRIEF DESCRIPTION OF THE DRAWINGS

FIG. 1 is a partially exploded assembly view demonstrating a coupling apparatus for a rotary surgical tool;
FIG. 2 is a partially exploded assembly view demonstrating a cutaway portion of a receiving coupler of a coupling apparatus;
FIG. 3A is a front, partial assembly view demonstrating a locking collar of a coupling apparatus in a locked position;
FIG. 3B is a front, partial assembly view demonstrating a locking collar of a coupling apparatus in an unlocked position;
FIG. 4A is a side cross-sectional assembly view demonstrating an assembly operation between a drive head and a receiving coupler;
FIG. 4B is a side cross-sectional assembly view demonstrating an assembly operation between a drive head and a receiving coupler;
FIG. 5 is a perspective view demonstrating a shank of a drive head selectively engaged with a receiving coupler of the coupling apparatus;
FIG. 6 is a partially exploded assembly view of a receiving coupler demonstrating a locking collar and release actuator of a coupling apparatus;
FIG. 7A is a front perspective view of a locking collar of a coupling apparatus;
FIG. 7B is a rear perspective view of a locking collar of a coupling apparatus;
FIG. 8A is a side assembly view of a coupling apparatus;
FIG. 8B is a front cross-sectional view of the coupling apparatus of FIG. 8A, extending along section line A-A, demonstrating a spring-bias arrangement of a release actuator and a locking collar;
FIG. 9A is a side assembly view of a coupling apparatus; and
FIG. 9B is a front cross-sectional view of the coupling apparatus of FIG. 8A, extending along section line B-B, demonstrating an exemplary arrangement of a release actuator and a locking collar in accordance with the disclosure.

### DETAILED DESCRIPTION

In the following description, reference is made to the accompanying drawings, which show specific implementations that may be practiced. Wherever possible, the same reference numbers will be used throughout the drawings to refer to the same or like parts. It is to be understood that other implementations may be utilized, and structural and functional changes may be made, without departing from the scope of this disclosure.

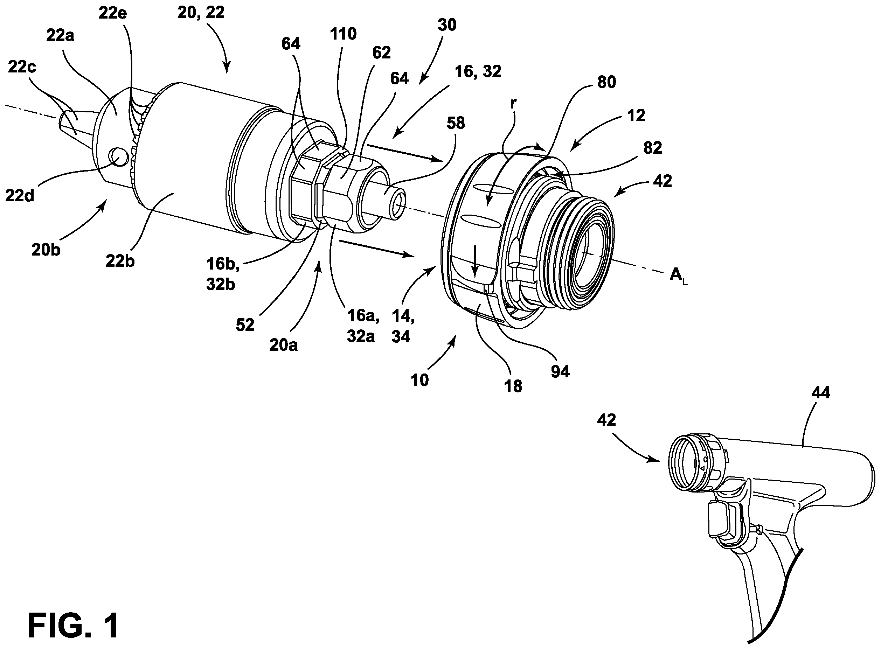

Referring generally to FIGS. 1-8B, various exemplary features and implementations of coupling apparatus 10 are shown. In various implementations, the coupling apparatus 10 may include a receiving coupler 12, including at least one socket 14 configured to receive a shank 16 of a drive head 20. In operation, the shank 16 of the drive head may be selectively engaged and disengaged from the receiving coupler 12 via a rotation "r" or interaction with a release actuator 18. As shown, the rotation r may be applied in a clockwise or counterclockwise direction about a longitudinal axis A_{L} of the coupling apparatus 10. The reversible rotation of the release actuator 18 may be implemented in combination with a symmetrical design, such that the coupling apparatus 10 may provide for ambidextrous operation by either right or lefthanded users. In this way, the disclosure may provide for an improved coupling apparatus 10 configured to suit a wide variety of applications.

As shown, the drive head 20 may correspond to a drill chuck 22 comprising a body 22a and a sleeve 22b configured to selectively clamp a drill bit or rotary tool within a plurality of jaws 22c. In this configuration, a chuck key may be used to engage a keyhole 22d and a plurality of gear teeth 22e of the sleeve 22b to clamp and release the jaws 22c from various rotary tools. Though discussed primarily in reference to the chuck 22, the coupling apparatus 10 may provide for quick and secure connection of various drive heads 20, rotary tools and accessories, or similar surgical tools for improved compatibility in operation. For example, the drive head 20 may comprise a variety of drive assemblies, linkages, etc. that may facilitate the operation of reciprocating, vibratory, oscillating, or various power tool accessories that may be driven by the drive assembly 42 of the rotary power tool 44. Accordingly, the disclosure may provide for an improved drive interface 30 that may suit a variety of applications.

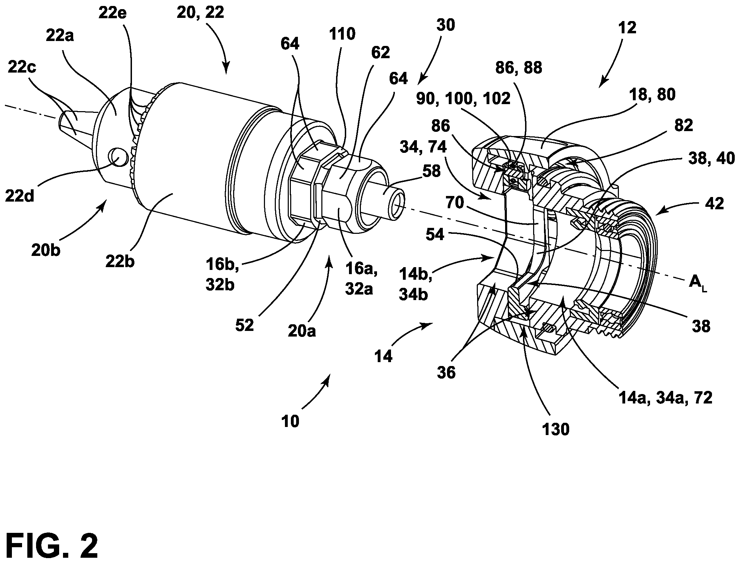

As best shown in FIGS. 1 and 2, the shank 16 of the drive head 20 may form an engaging profile 32 configured to mate with a receiving profile 34 formed by the at least one socket portion 14 of the receiving coupler 12. In operation, the engaging profile 32 may comprise one or more alignment features or flats that rotationally orient the drive head 20 relative to the receiving coupler 12 about a longitudinal engagement axis A_{L}. In general, the engaging profile 32 formed by the shank 16 may comprise a splined configuration, a keyed configuration, one or more flats as exemplarily illustrated, or similar configurations that may rotationally align the engaging profile 32 of the shank 16 with the receiving profile 34 of the coupler 12. Accordingly, the coupling apparatus 10 may be implemented in a variety of configurations without departing from the spirit of the disclosure.

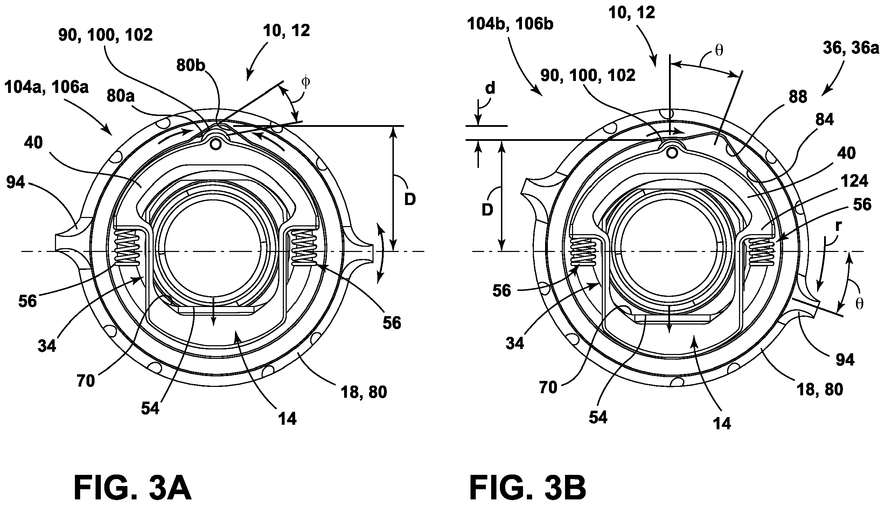

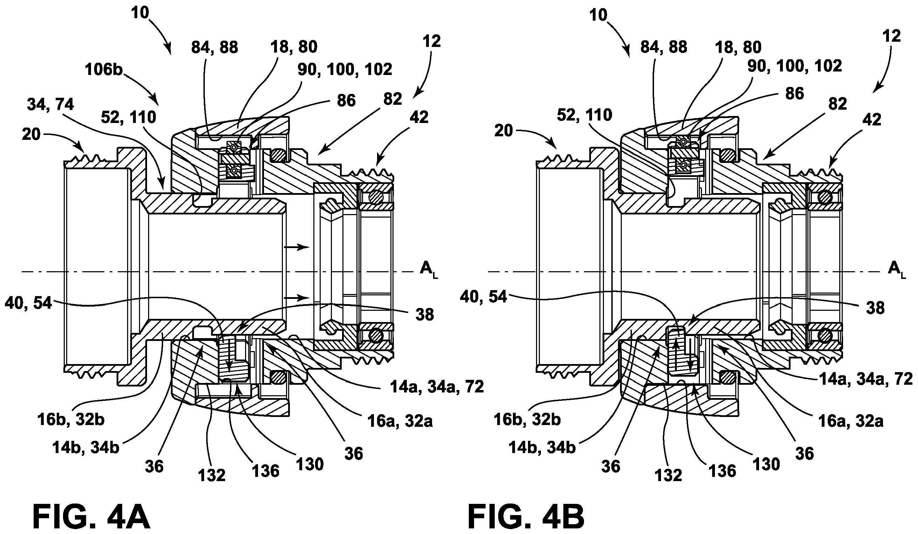

Still referring to FIGS. 1 and 2, the engaging profile 32 and the receiving profile 34 may include one or more rotational alignment portions 36 and a longitudinal engagement portion 38 that may longitudinally bind the shank 16 within the receiving coupler 12 along the longitudinal engagement axis A_{L}. In the example shown, the rotational alignment portions 36 of the receiving coupler 12 may comprise a first socket portion 14a and a second socket portion 14b. The first socket portion 14a may be positioned proximally of the second socket portion 14b and a locking collar 40 relative to a proximal drive assembly 42 of a rotary power tool 44. The first socket portion 14a may be configured to receive a corresponding first shank portion 16a forming a proximal end portion 20a of the drive head 20. Distal of the first shank portion 16a along the engagement axis A_{L}, a second shank portion 16b may form an engaging profile 32 that may be aligned with the second socket portion 14b in an assembled configuration 50, shown in FIGS. 3 and 4. Between the first shank portion 16a and the second shank portion 16b, a locking groove 52 may be formed and configured to receive a corresponding locking feature or engaging bar 54 formed by the locking collar 40. In this configuration, the first and second shank portions 16a, 16b may be received by the first and second socket portions 14a, 14b to align the drive head 20 with the receiving coupler 12 along the longitudinal engagement axis A_{L}. Once aligned in the assembled configuration 50, the locking bar 54 formed by the locking collar 40 may engage the locking groove 52 via a spring assembly 56 to retain the shank 16 of the drive head 20 in connection with the receiving coupler 12. In this configuration, a drive shaft 58 of the drive head 20 may be rotationally engaged with an output shaft (not shown) in connection with a drive motor of the power tool 44.

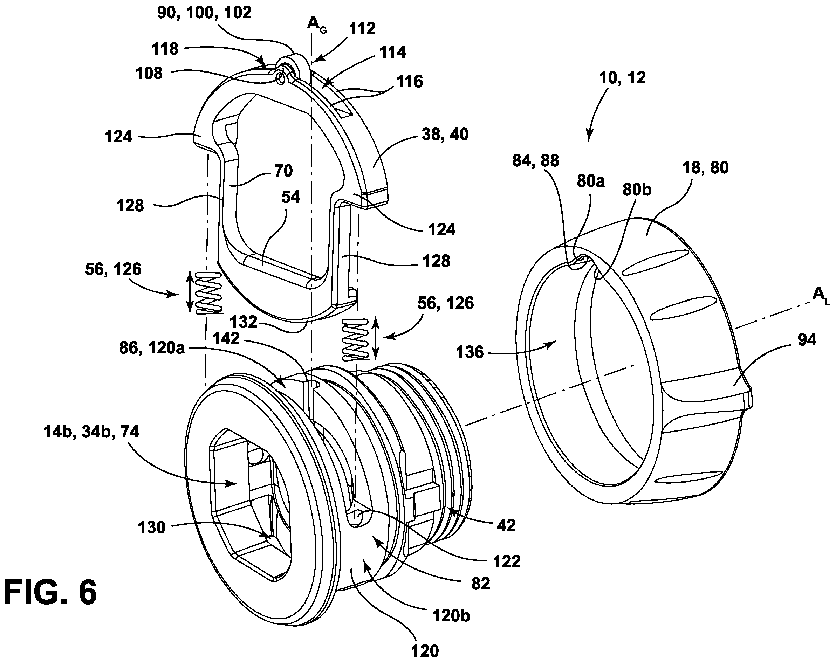

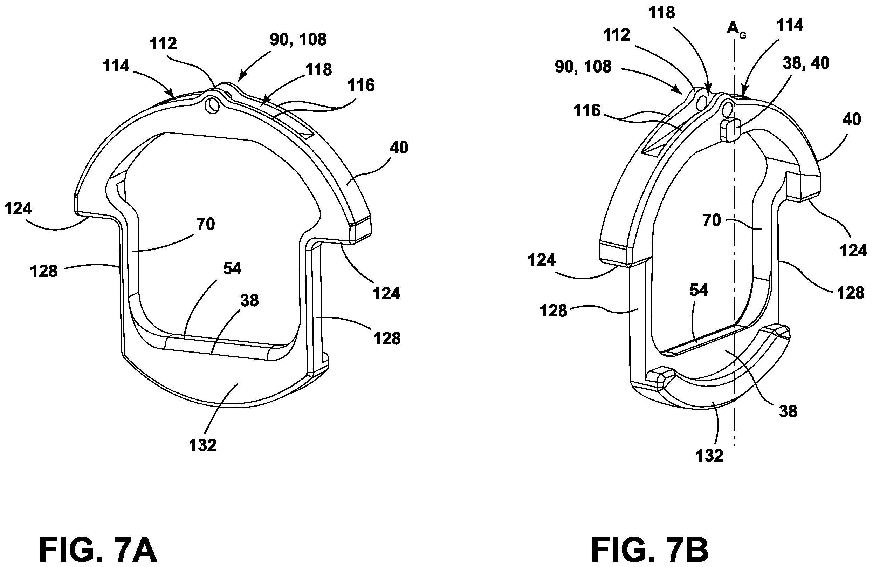

As best demonstrated in FIG. 2, the first shank portion 16a of the drive head 20 may generally form a cylindrical perimeter 62 forming a first profile portion 32a of the engaging profile 32. The first portion 32a may further comprise one or more circumscribed, cutaway, or flattened segments 64, which may be azimuthally distributed about the cylindrical perimeter 62. Upon mating the shank 16 with the receiving coupler 12, one or more of the flattened segments 64 may serve as rotational interlocking features to align an interior locking profile 70 formed by the locking collar 40 with the engaging profile of the shank 16 as later discussed and illustrated in reference to FIGS. 6 and 7. In this configuration, the first shank portion 16a may be aligned with the longitudinal engagement axis A_{L} formed by the receiving coupler 12 within a cylindrical opening 72 formed by an interior wall of the first socket portion 14a.

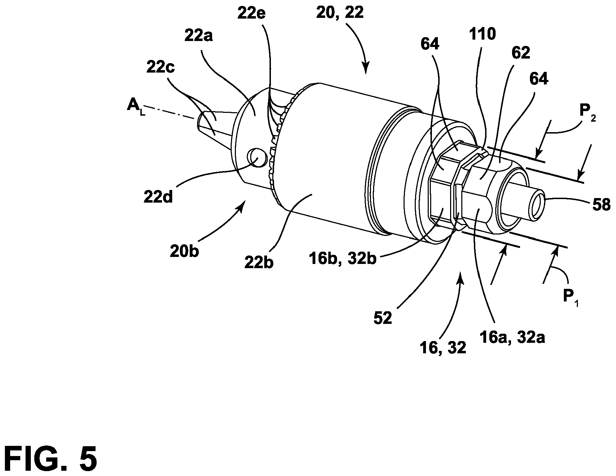

The second socket portion 14b of the receiving coupler 12 may be positioned distal of the first socket portion 14a and the locking groove 52 along the longitudinal axis A_{L}. Accordingly, the longitudinal axis A_{L} may correspond to a rotational engagement axis between the receiving coupler 12 and the shank 16. When assembling the drive head 20 with the receiving coupler 12, the first shank portion 16a may pass into a distal receiving opening 74 that may define a second portion 34b of the receiving profile 34. In the assembled configuration, a second profile portion 32b of the engaging profile 32 may engage the second portion 34b of the receiving profile 34 and rotationally or azimuthally align the shank 16 about the longitudinal axis A_{L} with the interior locking profile 70 of the locking collar 40. As best demonstrated in FIGS. 2 and 5, the second shank portion 16b may generally form a trapezoidal shape that may define the second portion 32b of the engaging profile 32. In the example provided, the second portion 32b may correspond to a rectangular exterior profile with chamfered corners or an irregular octagonal shape. However, it shall be understood that various perimeter profile shapes with flat segments, trapezoidal shapes or irregular profiles including one or more rounded exterior profile surfaces may similarly be implemented. In general, the shape of the second portion 32b of the engaging profile 32 may be received by a complimentary profile shape of the second portion 34b of the receiving profile 34. When arranged in the assembled configuration 50, the second portion 32b of the engaging profile 32 may axially align the shank 16 of the drive head 20 within the receiving coupler 12 along the longitudinal axis A_{L} and restrict rotation of the drive head 20 relative to the receiving coupler 12.

With the shank 16 inserted into the receiving coupler 12, each of the first and second shank portions 16a, 16b may be aligned with the corresponding first and second socket portions 14a, 14b along the longitudinal axis A_{L}. The shank 16 being inserted into the receiving coupler 12 and the resulting alignment in the assembled configuration 50 is illustrated in FIGS. 4A and 4B, respectively. Once aligned in the assembled configuration 50, the locking groove 52 interposed between the shank portions 16a, 16b may receive the deflected locking bar or locking detent 54 formed by the locking collar 40 in response to the spring force applied by the spring assembly 56. As previously discussed, the deflection of the locking bar 54 and corresponding deflection of the locking collar 40 may be controlled via an actuation or rotation of the release actuator 18 shown in FIGS. 3A and 3B. As shown, a sleeve 80 of the release actuator 18 is in connection with an exterior cylindrical profile 82 of the receiving coupler 12 and enclosed about a perimeter of the locking collar 40. In this configuration, a ramp portion 84a formed within an interior sleeve wall 84 of the sleeve 80 may engage the locking collar 40 to withdraw the locking bar 54 from the locking groove 52 by compressing the spring assembly 56 and displacing the locking profile 70 from obstructing a portion of the receiving profile 34.

As best illustrated and later discussed in reference to FIG. 6, the locking collar 40 may be disposed in a lateral opening 86 formed in a body of the receiving coupler 12 transverse to the longitudinal axis A_{L}. As shown in FIGS. 3 and 4, an interior sleeve wall 84 of the sleeve 80 may be enclosed about the locking collar 40 and the receiving coupler 12 aligning an engagement surface 88 of the interior sleeve wall 84 with a lateral positioning surface 90 formed by or in connection with the locking collar 40. As demonstrated in FIGS. 3A and 3B, an applied rotation "r" to the sleeve 80, for example via one or more wings or perimeter engagement features 94, may result in a displacement "d" of the locking collar 40 as a result of an engagement between the locking collar 40 and one of a plurality of opposing ramp portions 84a, 84b of the interior sleeve wall 84 formed on opposing sides of an apex 84c or peak. The opposing ramp portions 84a, 84b may provide for both clockwise and counterclockwise operation of the release actuator 18. As a result of the displacement d of the locking collar 40, the interior locking profile 70 formed by the locking bar 54 may be translated out of the locking groove 52 of the shank 16 laterally away from the longitudinal axis A_{L}. As discussed in further detail in reference to FIGS. 3A-4B, the translation of the locking collar 40 may provide for the effective locking and releasing of the drive head 20 from the receiving coupler 12 of the coupling apparatus 10 responsive to the applied rotation r.

Referring still to FIGS. 3-4, in various implementations, the lateral positioning surface 90 in connection with or formed by the locking collar 40 may be provided by a rotary element 100 that may be interposed between the locking collar 40 and the interior sleeve wall 84 forming the engagement surface 88. For example, the rotary element 100 may correspond to a bearing 102, bushing, or similar protruding engagement surface or element that receives an applied force as a result of the rotation r and the engagement with the one of the ramps 84a, 84b. The engagement with the ramp portions 84a, 84b may displace the locking collar 40 relative to the linear spring assembly 56. More specifically, the ramps 84a, 84b of the engagement surface 88 may be angled away from a tangential path oriented about the longitudinal axis A_{L}, such that a radial distance of the engagement surface 88 relative to the longitudinal axis A_{L} may vary as a result of a change in a rotation angle θ of the sleeve 80 relative to the receiving coupler 12. For example, the ramps 84a, 84b of the engagement surface 88 may be angled relative to a tangential path of the sleeve 80 about the longitudinal engagement axis A_{L} at an engagement angle Φ. The engagement angle Φ may vary based on the desired rotation angle θ of the sleeve 80 necessary to displace the locking collar 40 and release the shank 16. In some cases, the engagement angle Φ may vary from approximately 10° to 60° and in some implementations, may range from approximately 15° to 50° or 17° to 35°.

In operation, the applied rotation r to the perimeter or perimeter engagement features 94 of the sleeve 80 may result in a change in the radial distance "D" of the engagement surface 88 relative to the longitudinal axis A_{L} and resulting in the displacement distance d of the rotary element 100 in connection with the locking collar 40. An at rest position 104a may correspond to a locked configuration 106a of the coupling apparatus 10 with a rotation angle θ of approximately 0°, as demonstrated in FIG. 3A. Further, a compressed position 104b or release configuration 106b of the release actuator 18 with the sleeve 80 manually rotated to a rotation angle θ greater than 5° is illustrated in FIG. 3B. In various implementations, the throw or extent of the rotation angle θ necessary to effectuate the release position 106b from the locked position 106a may range from approximately, 10°, 20°, 30° or more. As demonstrated, the applied rotation r of the sleeve 80 may result in the compression of the spring assembly 56, causing the displacement distance d of the locking bar 54 of the locking collar 40 and withdrawal of the locking bar 54 from locking groove 52 formed within the engaging profile 32 of the shank 16.

As best demonstrated in FIGS. 6, 7A, and 7B, the rotary element 100 or bearing 102 may be connected to a protruding tab 108 that may be formed at an apex 112 of the locking collar 40. The protruding tab 108 may comprise a lateral slot 114 formed by opposing sidewalls 116 spaced along the longitudinal axis A_{L} in connection with fore and aft portions of the locking collar 40. The opposing sidewalls 116 may form a connection channel 118 centrally located on the locking collar 40 between the laterally extending support arms 124 or support wings. In this configuration, the connection channel 118 may be integrally formed by the locking collar 40, such that the rotary element 100a may be connected between the support arms 124 to rotate freely along the ramped surfaces 84a, 84b lateral to the longitudinal axis A_{L}.

Referring now to FIG. 5, the exemplary drive head 20 in the form of the drill chuck 22 is described in further detail. As shown, the shank portions 16a, 16b may form successive portions of the coupling or drive interface 30 extending along the longitudinal axis A_{L}. As previously discussed, the first and second shank portions 16a, 16b may comprise differing first and second engaging profiles 32a, 32b. The cylindrical perimeter 62 of the first shank portion 16a may comprise a first profile clearance dimension P₁, and the exemplary trapezoidal or octagonal second engaging profile 32b of the second shank portion 16b may comprise a second profile clearance dimension P₂. A difference between the profile clearance dimensions P₁ and P₂ may form a stepped intermediate wall 110 extending from the locking groove 52 to the second engaging profile 32b of the second shank portion 16b. In this configuration, the stepped intermediate wall 110 may form a positive stop aligning the shank 16 along the longitudinal axis A_{L} within the socket 14 of the receiving coupler 12.

As previously discussed, each of the shank portions 16a, 16b may comprise one or more flattened segments 64. In reference to the first shank portion 16a, the flattened segments 64 may be azimuthally distributed about the cylindrical perimeter 62. Similarly, the flattened segments 64 may correspond to flat or straight profile segments of the chamfered rectangular or octagonal second engaging profile 32b. In the assembled configuration 50, one or more of the flattened segments 64 or irregular segment of the shank portions 16a, 16b may rotationally align the shank 16 within the receiving coupler 12. Further, the locking groove 52 may have corresponding flattened segments 64 that may be aligned with the corresponding flattened segments 64 formed in the first and second shank portions 16a, 16b. In an exemplary implementation, each of the flattened segments 64 may be azimuthally aligned about the longitudinal axis A_{L}, such that the corresponding surfaces formed by the interior locking profile 70 of the locking collar 40 and the second receiving profile portion 34b of the second socket portion 14b are aligned in correspondence about the longitudinal axis A_{L}. As best demonstrated in FIG. 2, the cylindrical opening 72 formed by the first receiving profile 34a of the first socket portion 14a may align within the corresponding cylindrical perimeter 62 of the first shank portion 16a. Accordingly, the receipt of the first shank portion 16a within the first socket portion 14a may provide limited alignment of the longitudinal axis A_{L} to assist in insertion of the shank 16. Such a configuration may prevent unnecessary confinement of the shank 16 within the receiving coupler 12 and improve both manufacturability as well as the assembly of the components forming the coupling apparatus 10.

Though discussed primarily in reference to the chuck 22, the coupling apparatus 10 may provide for quick and secure connection of various drive heads 20, rotary tools, surgical accessories, and/or similar surgical tools for improved compatibility in operation. For example, various dedicated tools may similarly be implemented in the form of drivers, drills, boring tools, and various rotary tools. Additionally, the drive head 20 may comprise additional drive assemblies, linkages, etc. that may facilitate the operation of reciprocating, vibratory, oscillating, or various other power tool accessories that may be driven by the rotary drive assembly 42 of the power tool 44.

Referring now to FIGS. 6 and 7, exemplary components of the coupling apparatus 10 are shown demonstrating further detail. As demonstrated in FIG. 6, the locking collar 40 may engage the lateral opening or slot 86 formed through a body of the receiving coupler 12 perpendicular to the longitudinal engagement axis A_{L}. The lateral opening 86 or slot may correspond to a lateral passage extending through an exterior surface of the receiving coupler 12 approximately through a first hemisphere 120a of a generally tubular, cylindrical body 120 forming the receiving coupler 12. In this configuration, the spring assembly 56 may correspond to a plurality of linear or coil springs that may be partially housed within corresponding apertures 122 or blind holes that may be formed in opposing side walls of the tubular, cylindrical body 120 to a depth configured to retain and/or align the springs with laterally extending support arms 124 formed on opposing sides of the locking collar 40. In this configuration, the spring assembly 56 may apply a spring force 126 approximately tangential to the tubular, cylindrical body 120 of the receiving coupler 12. The spring force may bias the locking collar 40 and corresponding locking groove 52 to impinge upon at least a portion of the receiving profile 34 in the at rest configuration 104a or locked position 106a as previously discussed.

Still referring to FIGS. 6 and 7, when inserted into the lateral opening 86 or slot, opposing parallel walls 128 of the locking collar 40 may extend between approximately opposing sides formed by a second hemisphere 120b of the tubular, cylindrical body 120. In this configuration, the opposing parallel walls 128 may extend through the second hemisphere 120b and into a perimeter or passthrough opening 130 formed through the tubular, cylindrical body 120 opposite the lateral opening 86. A perimeter wall 132 of the locking collar 40 may extend between the opposing parallel walls 128 and support the locking bar 54 perpendicular to the longitudinal engagement axis A_{L} and within the perimeter opening 130. As demonstrated, a profile shape of the perimeter wall 132 may approximately align with the adjacent profile of the tubular, cylindrical body 120.

As shown and discussed previously in reference to FIGS. 3A and 3B, the lateral positioning surface 90, which may correspond to the rotary element 100 or bearing 102, may be connected to the locking collar 40 opposite the locking bar 54 across the interior locking profile 70. Between the lateral support arms 124, the perimeter wall 132 may similarly align with the exterior profile of the tubular, cylindrical body 120 of the receiving coupler 12. In this configuration, a cylindrical opening 136 formed by the interior sleeve wall 84 may surround the tubular, cylindrical body 120 of the receiving coupler 12 and lock or bind the locking collar 40 to translate within the lateral opening 86 formed through the receiving coupler 12 over the displacement distance d. Accordingly, the displacement distance d may be limited by the relative radial distance D defined by the engagement angle Φ of the ramp portions 84a, 84b between the at rest configuration 104a and the compressed configuration 104b.

In addition to being bound within the sleeve 80 and the lateral opening 86, the translation of the locking collar 40 may be limited by an engagement between an alignment tab or protrusion 140 (see, FIG. 7B) formed by a portion of the perimeter wall 132 proximate to the perimeter engagement feature 94 or rotary element 100 and a corresponding channel 142 (see, FIG. 6) or trench formed perpendicular to the longitudinal axis A_{L} along a lateral guide axis A_{G}. In this configuration, the movement of the locking collar 40 within the lateral opening 86 and the sleeve 80 may be restricted to axial motion along the lateral guide axis A_{G} based on the interaction between the protrusion 140 and the channel 142. Accordingly, the biasing motion and corresponding displacement d of the locking collar 40 may be controlled or restricted to occur along the lateral guide axis A_{G} in response to the force applied by the lateral positioning surface 90 of the sleeve 80 as a result of the applied rotation r and resulting change in the rotation angle θ.

As previously discussed, the ramp portions 84a, 84b forming the lateral positioning surface 90 of the interior sleeve wall 84 may correspond to a profile shape that is symmetric across the lateral guide axis A_{G} or more generally related to a positive or negative rotation angle θ. In this configuration, the applied rotation r to the sleeve 80 may be applied in a clockwise or counterclockwise direction about the longitudinal axis A_{L} and provide for the same corresponding change in the radial distance D along the engagement angle Φ. The symmetric relationship of the features and the resulting displacement d regardless of the direction of rotation r may be particularly beneficial because the interaction with the coupling apparatus 10 may be similarly achieved for both righthanded and lefthanded users. For example, a lefthanded user may have a preference of turning the sleeve 80 in a counterclockwise direction, while a righthanded user may prefer to turn the sleeve 80 in a clockwise direction. The results of either corresponding change in the rotation angle θ may provide for the same displacement d of the locking bar 54 and release of the drive head 20.

Still referring to FIGS. 6 and 7, the perimeter engagement features 94 and the reversible disengagement of the locking bar 54 by both positive and negative rotation angles θ may similarly provide for one-handed use or engagement of the coupling apparatus by both lefthanded and righthanded users. For example, if a user were to reach an index finger along a barrel of the power tool 44, the fingertip may reach the perimeter engagement feature 94 on opposing sides of the sleeve 80. By gripping a handle of the power tool 44, a user may achieve the clockwise or counterclockwise change in the rotation angle θ to effectuate the release of the drive head 20. Once the applied rotation r is released, the spring force 126 associated with the spring assembly 56 may apply pressure to the lateral positioning surface 90, such that the force applied against the ramp portion 84a or 84b results in a rotation of the sleeve 80 back to the at rest position 104a or locked position 106a. In some implementations, the release actuator 18 may comprise a plurality of spring assemblies 150 that may further facilitate the actuation of the locking collar 40 or similar assemblies as discussed herein.

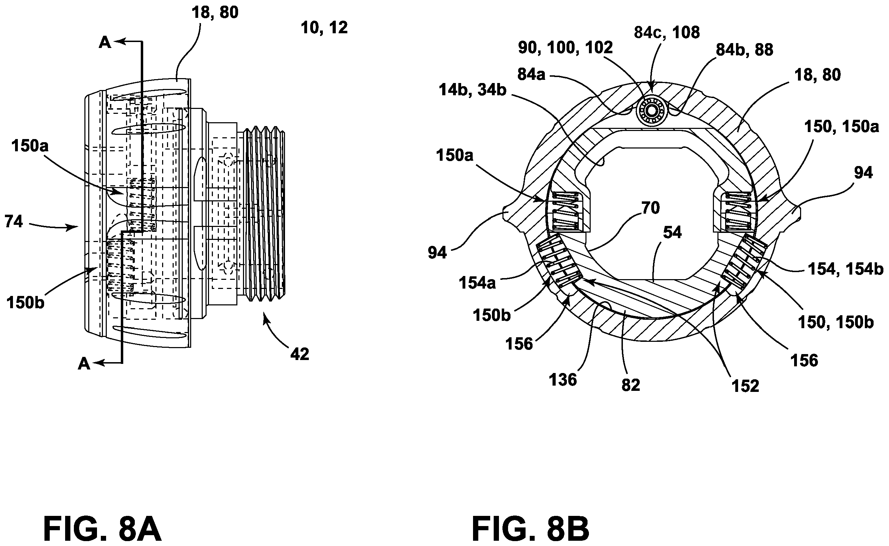

Referring now to FIGS. 8A and 8B, in some implementations, the coupling apparatus 10 may comprise a plurality of spring assemblies 150. The spring assemblies 150 are best shown in FIG. 8B, which is a compound cross-sectional view taken along section line A-A in FIG. 8A. As shown, a first spring assembly 150a may correspond to or be implemented similar to the spring assembly 56. Accordingly, the first spring assembly 150a may provide for the biasing spring force 126 opposing the translation locking bar 54 of the locking collar 40 applied to the support arms 124 along the lateral guide axis A_{G}. The second spring assembly 150b may correspond to a rotary or rotational spring assembly 152. In the example shown, the rotational spring assembly 152 may comprise opposing springs 154 disposed in openings 156 aligned between the interior sleeve wall 84 and an exterior surface of the second hemisphere 120b of the tubular, cylindrical body 120 formed by the receiving coupler 12. As best demonstrated by FIG. 8B, the opposing springs 154 may be retained in compression by interior surfaces formed within the opposing openings 156 between the sleeve 80 and the receiving coupler 12. In this configuration, the applied rotation r and resulting change in the rotation angle θ may alternately compress the opposing springs 154 depending on the direction of the rotation angle θ. For example, a counterclockwise rotation of the sleeve 80 may result in a compression of a first spring 154a and a clockwise rotation of the sleeve 80 may result in compression of a second spring 154b. In operation, the opposing springs 154 may provide for increased resistance to the applied rotation r that may be tuned to suit the operating characteristics of the coupling apparatus 10 for various applications in combination with the rotary tool 44.

The spring assemblies 150 are best shown in FIG. 8B, which is a compound cross-sectional view taken along section line A-A in FIG. 8A. As shown, a first spring assembly 150a may correspond to or be implemented similar to the spring assembly 56. Accordingly, the first spring assembly 150a may provide for the biasing spring force 126 opposing the translation locking bar 54 of the locking collar 40 applied to the support arms 124 along the lateral guide axis A_{G}. The second spring assembly 150b may correspond to a rotary or rotational spring assembly 152. In the example shown, the rotational spring assembly 152 may comprise opposing springs 154 disposed in openings 156 aligned between the interior sleeve wall 84 and an exterior surface of the second hemisphere 120b of the tubular, cylindrical body 120 formed by the receiving coupler 12. As best demonstrated by FIG. 8B, the opposing springs 154 may be retained in compression by interior surfaces formed within the opposing openings 156 between the sleeve 80 and the receiving coupler 12. In this configuration, the applied rotation r and resulting change in the rotation angle θ may alternately compress the opposing springs 154 depending on the direction of the rotation angle θ. For example, a counterclockwise rotation of the sleeve 80 may result in a compression of a first spring 154a and a clockwise rotation of the sleeve 80 may result in compression of a second spring 154b. In operation, the opposing springs 154 may provide for increased resistance to the applied rotation r that may be tuned to suit the operating characteristics of the coupling apparatus 10 for various applications in combination with the rotary tool 44.

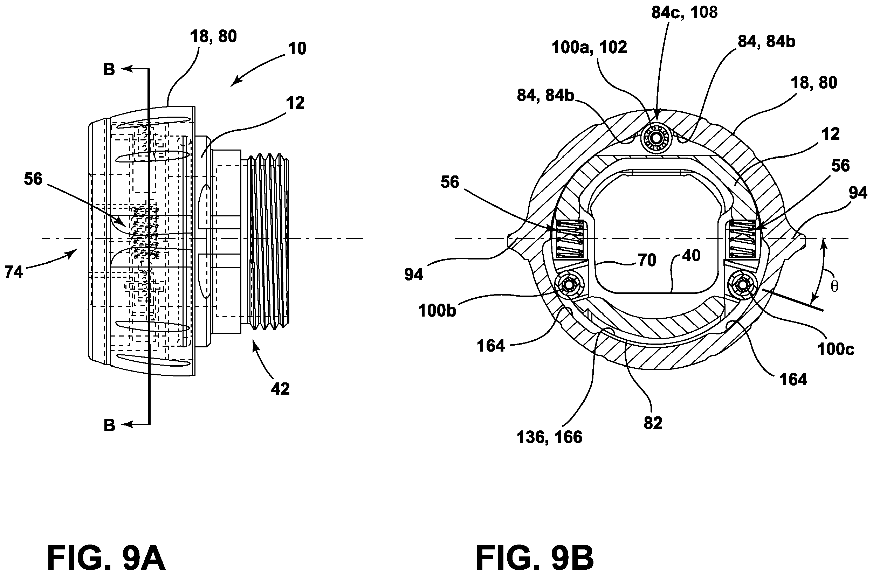

Referring now to FIGS. 9A and 9B, in some implementations, the coupling apparatus 10 may comprise a plurality of the rotary elements 100 or bearings 102. Though demonstrated in separate examples, it shall be understood that the coupling apparatus 10 may include the plurality of the rotary elements 100 and the plurality of spring assemblies 150 in combination. As shown, the rotary elements 100 may be angularly spaced about the longitudinal axis A_{L} of the receiving coupler 12. As previously demonstrated and described in reference to FIGS. 6, 7A, and 7B, the first rotary element 100a may be connected to the connection tab 108 formed at the apex 112 of the locking collar 40. The connection tab 108 may comprise the lateral slot 114 formed through the opposing sidewalls 116 forming the connection channel 118. One or more of a second rotary element 100b and a third rotary element 100c may be angularly distributed about the longitudinal axis A_{L} between the exterior cylindrical profile 82 of the receiving coupler 12 and at least one interior rotary support surface 164 formed on an interior sleeve surface 166 within the cylindrical opening 136 of the sleeve 80. In this configuration, the rotary elements 100a, 100b, 100c may be implemented as the bearings 102, bushings, rollers, etc. angularly distributed about the longitudinal axis A_{L} in a rotary hub configuration to provide for a smooth rotation of the sleeve 80 about the coupler 12.

In operation, the spring-biased position of the locking collar 40 may cause the first rotary element 100a to engage the apex 84c or peak of the interior sleeve wall 84 between the ramped portions 84a, 84b in response to the sleeve 80 orientated in a neutral position (e.g., a zero-degree rotation angle θ). In this position, the second and third rotary elements 100b, 100c may be interposed between the exterior cylindrical profile 82 of the receiving coupler 12 and the interior rotary support surface 164 formed by the interior sleeve surface 166. In this resting or locked configuration 106a (FIG. 3A), the engagement sleeve 80 may be supported by the rotary elements 100a, 100b, 100c, where the second and third rotary elements 100b, 100c generally oppose the pressure applied by the spring assembly 56 via the engagement of the first rotary element 100a with the interior sleeve wall 84. In operation, the second and third rotary elements 100b, 100c may continue to oppose the pressure applied by the spring assembly 56 to the interior sleeve wall 84 via the first rotary element 100a and provide for a smooth, rolling engagement between the sleeve 80 and the coupler 12.

As previously discussed, the rotation of the sleeve 80 over the rotation angle θ, may reposition the locking collar 40 from the locked configuration 106a (FIG. 3A) to the release configuration 106b (FIG. 3B). Throughout the rotation of the sleeve 80, the second and third rotary elements 100b, 100c may continue to oppose the increased spring force applied by the spring assembly 56 to the opposing side of the sleeve 80 via the locking collar 40. The plurality of rotary elements 100 may ensure that the sleeve 80 smoothly rotates about the receiving coupler 12 throughout the rotation angle θ about the longitudinal axis A_{L} from the locked configuration 106a (FIG. 3A) to the released configuration 106b (FIG. 3B). Once the external rotational force is released from the sleeve 80 (e.g., via the perimeter engagement feature 94), the compressed spring assembly 56 may expand or extend. The extension of the spring assembly 56 may cause the first rotary element 100a to engage the corresponding ramped portion 84a, 84b, resulting in the first rotary element 100a returning to the apex 84c of the interior sleeve wall 84 and the rotation angle θ returning to the resting or locked configuration 106a. As shown, the rotary elements 100a, 100b, 100c are approximately evenly spaced angularly about the longitudinal axis A_{L}. However, it shall be understood that the spacing and the number of rotary elements may vary while maintaining a smooth rotary motion of the sleeve 60 about the receiving coupler 12 as supported by the intermediate engagement of the plurality of rotary elements 100 therebetween.

As provided by the previously discussed examples, the disclosure provides for a coupling apparatus 10 that may provide for rapid and secure exchange of surgical accessories in connection a drive head 20. The features and operations of the coupling apparatus may provide for improved longevity by limiting wear components while also improving manufacturability. In various implementations, the coupling apparatus 10 may also improve user interaction for professionals with diverse preferences for operation. For example, in some cases, the coupling apparatus may provide for ambidextrous operation by engaging or releasing corresponding drive heads or accessories by either clockwise or counterclockwise rotation. The ambidextrous operation may also provide for the release of drive heads via a single finger engagement (e.g., an index or trigger-finger) that otherwise controls a drive control input of a power tool.

According to some aspects of the disclosure, a coupling apparatus for a surgical tool comprises a receiving coupler including a first socket portion and a second socket portion forming at least one receiving profile aligned with a rotational engagement axis of a drive head. A locking collar is interposed between the socket portions, and a release actuator is in connection with the locking collar, such that rotation of the release actuator relative to the socket about the engagement axis moves the locking collar relative to the engagement axis.

According to various aspects, the disclosure may implement one or more of the following features or configurations in various combinations:
- the movement of the locking collar in response to the rotation of the release actuator moves a locking surface of the locking collar away from the engagement axis;
- a roller disposed between a release collar and the locking collar, the release collar forming an exterior engagement surface of the release actuator configured to receive an external force causing the rotation;
- the release collar comprises a sleeve or tubular body comprising an interior engagement surface extending at an engagement angle relative to a cylindrical sleeve opening radially spaced about the engagement axis;
- the release collar forms an engagement surface forming a ramp that varies in a radial distance about the engagement axis in response to the rotation, and wherein the rotation of the sleeve engages the ramp translating the locking collar within the socket;
- the rotary element comprises a bearing in connection with the locking collar that rotates in response to the rotation of the release actuator and displaces the locking collar perpendicular to the engagement axis in response to an interaction with a changing radial position of the ramp about the engagement axis;
- the locking collar is biased into a locking configuration by at least one spring and impinges on the at least one receiving profile;
- the locking collar is disposed in a lateral opening formed in the socket and the at least one (translational) spring is interposed between the socket and the locking collar;
- the locking collar forms a locking surface that engages a locking groove formed in an exterior engagement surface of the drive head;
- the locking groove comprises a retention feature formed by the receiving coupler between the socket portions, and the locking collar forms a complementary engagement feature;
- the retention feature comprises a receiving flat that is engaged by a locking or an engaging bar extending perpendicular to the engagement axis and formed by an interior wall forming a movable opening of the locking collar;
- at least one of the first socket portion and the second socket portion is configured to receive the exterior engagement surface of the drive head;
- at least one of the first socket portion and a second socket portion comprises an interior alignment surface that engages a rotational alignment surface of the drive head and rotationally orients the drive head to the receiving coupler;
- the first socket portion is formed proximal to a receiving opening formed by the receiving coupler and the second socket portion is formed distal of the first socket portion;
- the first socket portion comprises a rotational alignment surface configured to rotationally align the drive head about the engagement axis and the second socket portion comprises a cylindrical opening uniformly about the engagement axis;
- the rotational alignment surface diverges from a tangential alignment about the engagement axis restricting a rotation of the drive head relative to the socket about the engagement axis;
- the rotational alignment surface comprises at least one interior flat defining the receiving profile and configured to receive a corresponding exterior flat formed by an engaging profile of the drive head;
- the rotational alignment surface comprises a plurality of alignment surfaces azimuthally distributed about the engagement axis;
- the drive head is in connection with a rotary tool comprising at least one of a chuck, a drill, a driver, and a rotary tool accessory adapter; and/or
- the receiving coupler is in connection with a rotary tool configured to rotate the drive head.

According to another aspect of the disclosure, a surgical accessory comprises a head forming an engagement axis extending from a working distal portion to a shank at a proximal engaging portion. The surgical accessory is configured to engage a receiving socket of a rotary tool and comprises a first shank portion of the shank forming a first engaging profile and a second shank portion of the shank forming a second engaging profile adjacent to the first shank portion. A locking groove is interposed between the first shank portion and the second shank portion. At least one alignment feature is disposed on the second shank portion. The alignment feature rotationally aligns the shank within a receiving profile of the receiving socket.

According to various aspects, the disclosure may implement one or more of the following features or configurations in various combinations:
- the alignment feature comprises a perimeter variation in the second engaging profile defined by a change in a perimeter distance of the second engaging profile rotationally about the engagement axis;
- the alignment feature engages a complementary shape of the receiving profile of the receiving socket rotationally orienting the shank to the receiving socket;
- the alignment feature comprises at least one flat segment formed by the second engaging profile along the second shank portion;
- the locking groove comprises a receiving notch rotationally aligned about the engagement axis with a locking profile of the receiving socket by the alignment of the alignment feature to the receiving socket;
- at least one alignment feature comprises a first flattened segment formed on the second shank portion and the receiving notch comprises a second flattened segment formed within the locking groove, wherein the first flattened segment and the second flattened segment are rotationally aligned about the engagement axis;
- the second shank portion is positioned distal of the first shank portion;
- the locking groove forms a locking channel between the first shank portion and the second shank portion, wherein the locking channel forms a receiving profile engaged by a locking collar within the receiving socket; and/or
- the locking collar circumscribes a portion of the first engaging profile and the second engaging profile.

According to yet another aspect of the disclosure, a coupling interface for a surgical tool comprises a shank of an interchangeable tool accessory forming a proximal engaging portion including a receiving groove. A receiving coupler receives the shank and forms at least one receiving profile extending about an engagement axis of the tool accessory. A locking collar is disposed in the receiving coupler, while a release actuator is in connection with the locking collar. The release actuator comprises at least one ramp surface that radially varies about the engagement axis along a ramp angle, wherein a rotation of the release actuator about the engagement axis angularly repositions the ramp surface about the engagement axis and displaces the locking collar.

According to various aspects, the disclosure may implement one or more of the following features or configurations in various combinations:
- the locking collar engages the groove in a locked configuration and the displacement of the locking collar moves the locking collar out of the groove in a released configuration;
- rotation of the release actuator displaces the locking collar in a clockwise direction and a counterclockwise direction;
- at least one ramp surface comprises a plurality of opposing ramp surfaces into which an engagement surface of the locking collar extends in the locked configuration;
- a roller disposed forming the engagement surface and disposed between the locking collar and at least one ramp surface; and/or
- the release actuator comprises a sleeve or tubular body comprising an interior engagement surface, wherein the at least one ramp is formed within a cylindrical sleeve opening of the sleeve along the ramp angle.

It will be understood that any described processes or steps within described processes may be combined with other disclosed processes or steps to form structures within the scope of the present device. The exemplary structures and processes disclosed herein are for illustrative purposes and are not to be construed as limiting.

It is also to be understood that variations and modifications can be made on the aforementioned structures and methods without departing from the concepts of the present device, and further it is to be understood that such concepts are intended to be covered by the following claims unless these claims by their language expressly state otherwise.

The above description is considered that of the illustrated embodiments only. Modifications of the device will occur to those skilled in the art and to those who make or use the device. Therefore, it is understood that the embodiments shown in the drawings and described above are merely for illustrative purposes and not intended to limit the scope of the device, which is defined by the following claims as interpreted according to the principles of patent law, including the Doctrine of Equivalents

## Claims

1. A coupling apparatus (10) for a surgical tool comprising:
a receiving coupler (12) comprising a first socket portion (14a) and a second socket portion (14b) forming at least one receiving profile (34) aligned with a rotational engagement axis (A_{L}) of a drive head (20);
a locking collar (40) interposed between the socket portions (14); and
a release actuator (18) in connection with the locking collar (40), wherein a rotation of the release actuator (18) relative to the socket (14) about the engagement axis (A_{L}) moves the locking collar (40) relative to the engagement axis (A_{L}).

2. The coupling apparatus (10) according to claim 1, wherein the movement of the locking collar (40) in response to the rotation of the release actuator (18) moves a locking surface of the locking collar (40) away from the engagement axis (A_{L}).

3. The coupling apparatus (10) according to any one of claims 1-2, further comprising:
a roller disposed between a release collar and the locking collar (40), the release collar forming an exterior engagement surface of the release actuator (18) configured to receive an external force causing the rotation.

4. The coupling apparatus (10) according to claim 3, wherein the release collar comprises a sleeve (80) or tubular body comprising an interior engagement surface extending at an engagement angle relative to a cylindrical sleeve opening radially spaced about the engagement axis (A_{L}).

5. The coupling apparatus (10) according to claim 3, wherein the release collar forms an engagement surface (88) forming a ramp (84) that varies in a radial distance about the engagement axis (A_{L}) in response to the rotation, and wherein the rotation of the sleeve (80) engages the ramp (84) translating the locking collar (40) within the socket (14).

6. The coupling apparatus (10) according to claim 5, wherein the rotary element (100) comprises a bearing (102) in connection with the locking collar (40) that rotates in response to the rotation of the release actuator (18) and displaces the locking collar (40) perpendicular to the engagement axis (A_{L}) in response to an interaction with a changing radial position of the ramp (84) about the engagement axis (A_{L}).

7. The coupling apparatus (10) according to any one of claims 1-6, wherein the locking collar (40) is biased into a locking configuration by at least one spring and impinges on the at least one receiving profile (34).

8. The coupling apparatus (10) according to any one of claims 1-7, wherein the locking collar (40) is disposed in a lateral opening (86) formed in the socket (14) and the at least one (translational) spring is interposed between the socket (14) and the locking collar (40).

9. The coupling apparatus (10) according to any one of claims 1-8, wherein the locking collar (40) forms a locking surface that engages a locking groove formed in an exterior engagement surface of the drive head (20).

10. The coupling apparatus (10) according to claim 9, wherein the locking groove (52) comprises a retention feature formed by the receiving coupler (12) between the socket portions (14a, 14b), and the locking collar (40) forms a complementary engagement feature (94).

11. The coupling apparatus (10) according to claim 10, wherein the retention feature comprises a receiving flat that is engaged by a locking or an engaging bar (54) extending perpendicular to the engagement axis (A_{L}) and formed by an interior wall forming a movable opening of the locking collar (40).

12. The coupling apparatus (10) according to claim 9. wherein at least one of the first socket portion and the second socket portion is configured to receive the exterior engagement surface of the drive head.

13. The coupling apparatus (10) according to claim 12, wherein at least one of the first socket portion (14a) and a second socket portion (14b) comprises an interior alignment surface that engages a rotational alignment surface of the drive head (20) and rotationally orients the drive head (20) to the receiving coupler (12).

14. The coupling apparatus (10) according to claim 12, wherein the first socket portion (14a) is formed proximal to a receiving opening formed by the receiving coupler (12) and the second socket portion (14b) is formed distal of the first socket portion (14a).

15. The coupling apparatus (10) according to claim 12, wherein the first socket portion (14a) comprises a rotational alignment surface configured to rotationally align the drive head (20) about the engagement axis and the second socket portion (14b) comprises a cylindrical opening (72) uniformly about the engagement axis.
